# EUROPEAN PATENT APPLICATION

(11) **EP 1 254 640 A2**
(43) Date of publication of application: **06.11.2002**
(21) Application number: 02253116.4
(22) Date of filing: 02.05.2002
(51) Int. Cl.: A61B 17/70

(54) **Swivel coupling**

(30) Priority: 02.05.2001 GB 0110814
(71) Applicant: Biomet Merck Limited, Bridgend, South Glamorgan CF31 3XA (GB)
(72) Inventor: Truscott, James William, SN2 7QD (GB)
(74) Representative: Giles, Ashley Simon

(57) **Abstract**

A swivel coupling (1) for attachment to human or animal bone comprises a main body (2) consisting of first and second clamping elements (4, 6) which are clamped together by means of a threaded fastener (10). A first connection element comprising a substantially spherical annular coupling (24) is located between the clamping elements (4, 6) and in a relaxed state of the threaded fastener (10) is able to swivel relative to the main body (2). When the threaded fastener (10) is tightened, the clamping elements (4, 6) are brought together to clamp the connection element (24) in a fixed position relative to the main body (2). A bone engaging element such as a bone screw or hook (32), or a spinal correction rod may be fitted to the connection element (24). In a preferred embodiment, two connection elements (24, 70) are provided, both of the connection elements (24, 70) being clamped when the threaded fastener (10) is tightened. In this preferred embodiment, a bone screw or hook (32) may be fitted to one of the connection elements (24) and a spinal correction rod may be fitted to the other connection element (70).

## Description

### BRIEF DESCRIPTION OF THE INVENTION

This invention relates to a swivel coupling for attachment to human or animal bone.

### BACKGROUND TO THE INVENTION

It is well known to stabilise a human spine by means of a spinal correction rod which is fixed to two or more vertebrae. The Webb-Morley spinal correction system is a well known and successful system for stabilising the spine and comprises a spinal correction rod to which are fitted bone engaging elements comprising bone screws or hooks. In order to connect the bone engaging elements to the spinal correction rod, the spinal correction rod is located in a slot formed in the head of the bone engaging element. A collet is placed in the slot, above the spinal correction rod, and a threaded ring is screwed down onto the collet, thereby trapping the spinal correction rod between the collet and the base of the slot. The system is very flexible, since it allows repeated readjustment of the position of the bone engaging elements relative to the spinal correction rod. Adjustment is possible by moving the bone engaging element along the length of the rod and also by rotating the bone engaging element around the rod, until the required angle and position is found.

In certain circumstances, it would also be useful to able to angle the bone engaging element within a plane coincident with or parallel to the plane of the spinal correction rod. The present invention has been developed with this in mind.

### SUMMARY OF THE INVENTION

According to a first aspect of the present invention there is provided a swivel coupling for attachment to human or animal bone, the device comprising a main body and a first connection element, the main body comprising first and second clamping elements and tightening means for tightening the clamping elements together, the first connection element being situated between the clamping elements with some clearance, so that in a relaxed state of the tightening means, the first connection element is able to swivel relative to the body portion, whereas when the tightening element is tightened, the clamping elements are brought together to clamp the first connection element in a fixed position relative to the body portion.

Preferably, the first and second clamping elements are fixed together at an end of the main body. Alternatively, the first and second clamping elements may be fixed together at both ends of the main body and are spaced apart in between the said ends.

Preferably, the first and second clamping elements are integrally formed.

Preferably, the fist connection element is at least partially spherical.

Preferably, the tightening means comprises a screw, which is engaged in a thread formed in the second clamping element, and which bears on the first clamping element with the underside of its head.

Preferably, a second connection element is situated between the first and second clamping elements and is clamped in position relative to the main body and the first connection element when the tightening element is tightened.

The tightening element is preferably situated between the first connection element and the second connection element.

Preferably, the first connection element is provided with an opening which receives a first substantially tubular member. The first substantially tubular member preferably comprises a bone engaging element.

Preferably, the second connection element is provided with a second opening which receives a second substantially tubular member. Preferably the second substantially tubular member comprises a spinal correction rod.

Preferably, a central axis of the opening in the first connection element is not parallel to a central axis of the opening in the second connection element.

For example, the central axis of the opening in the first connection element may be substantially perpendicular to the central axis of the opening in the second connection element.

Preferably, the second connection element is split and/or slotted, so that as the clamping elements are tightened onto the second connection element, the opening through the second connection element is reduced in diameter, thereby to clamp the second substantially tubular member.

Preferably, the second connection element is provided with a plurality of slots which extend from the central opening to the outer surface of the second connection element.

Preferably, the second connection element is at least partially spherical.

According to a second aspect of the present invention there is provided a device for attachment to human or animal bone comprising a bone engaging element and a connection element, the bone engaging element comprising a split collet, the open end of the collet being provided with an at least partially tapered bore in which is a located a threaded element, the split collet being received within an opening formed in the connection element, the threaded element being screwed into the open end of the collet to splay the collet into contact with an internal wall of the opening formed in the connection element.

### Brief Description of the Drawings

For a better understanding of the present invention and to show how the same may be carried into effect reference will now be made, by way of example, to the accompanying drawings, in which:-
Figure 1 is a top view of a swivel joint coupling in accordance with the present invention;
Figure 2 is a cross-section through a swivel joint coupling and bone engaging hook in accordance with a first embodiment of the present invention;
Figure 3 is a side view of a part-spherical second connection element; and
Figure 4 is a view from above of the second connection element of Figure 5;
Figure 5 is a view from above of a second embodiment of swivel coupling;
Figure 6 is a cross-section through a second embodiment of swivel coupling.

### Detailed Description of Preferred Embodiments

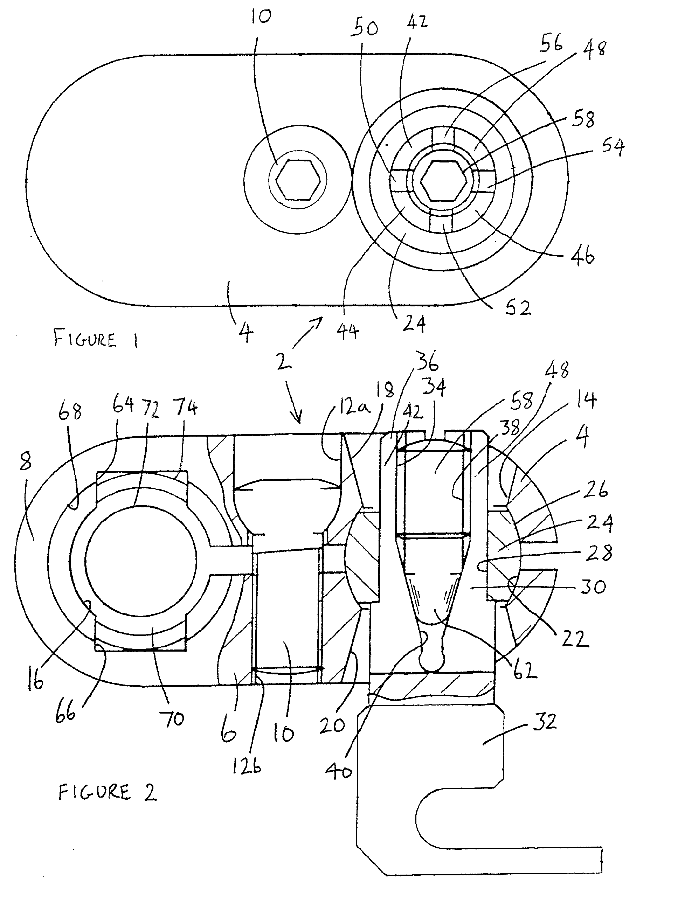

Referring to Figures 1 and 2, a swivel coupling 1 in accordance with the first embodiment of the present invention comprises a main body 2 which is machined from stainless steel or titanium or any other medically acceptable material with appropriate mechanical properties. The body portion 2 is divided into first and second clamping elements 4, 6 which are interconnected at a first end 8 of the main body 2.

A tightening element in the form of a machine screw 10 is located in aligned openings 12a, 12b formed through the first clamping element 4 and second clamping element 6 respectively. The first opening 12a comprises a counter sink which receives the head of the machine screw 10 and the second opening 12b comprise a threaded bore into which the threads of the machine screw 10 engage.

Two large cavities 14, 16 are formed in the main body 2 on opposite sides of the openings 12a, 12b. The first cavity 14 comprises a pair of conical openings 18, 20 which taper inwardly towards a central spherical region 22. A connection element comprising an annular coupling 24 is located in the central spherical regional 22 of the first cavity 14. The radially outer surface 26 of the annular coupling 24 is spherical and fits within the central spherical region 22 of the first cavity 14 with sufficient clearance to allow the annular coupling 24 to swivel relative to the main body, when the machine screw 10 is loose.

An opening 28 is formed through the annular coupling 24. The opening 28 receives a collet portion 30 of a bone engaging element in the form of a bone hook 32. A bore 34 is formed in an open end 36 of the collet portion 30. The bore 34 comprises a threaded bore portion 38 which opens into a smooth walled tapered portion 40.

The collet 30 is split into four identical tangs 42, 44, 46, 48 by four equidistantly spaced slots 50, 52, 54, 56 which extend from the open end 36 of the collet portion 30 to a position midway along the length of the tapered portion 40 of the bore 34. A grub screw 58 engages in the threaded portion 38 of the bore 34. The grub screw 58 has a tapered nose portion 62 which cooperates with the tapered portion 40 of the bore 34.

The second cavity 16 comprises a pair of substantially rectangular recesses 64, 66 which extend through the full width of the main body 2 in a direction substantially perpendicular to a central axis of the machine screw 10. The recesses 64, 66 open into a central spherical region 68 of the cavity 16. A second connection element comprising an annular coupling 70 is housed within the central spherical region 68.

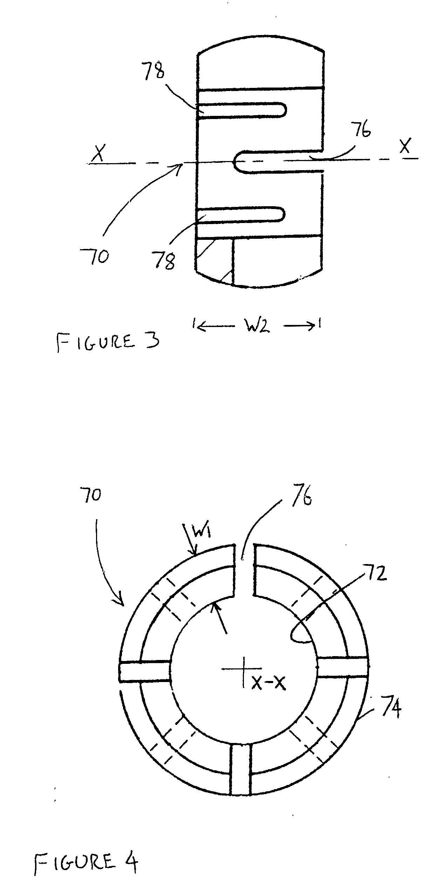

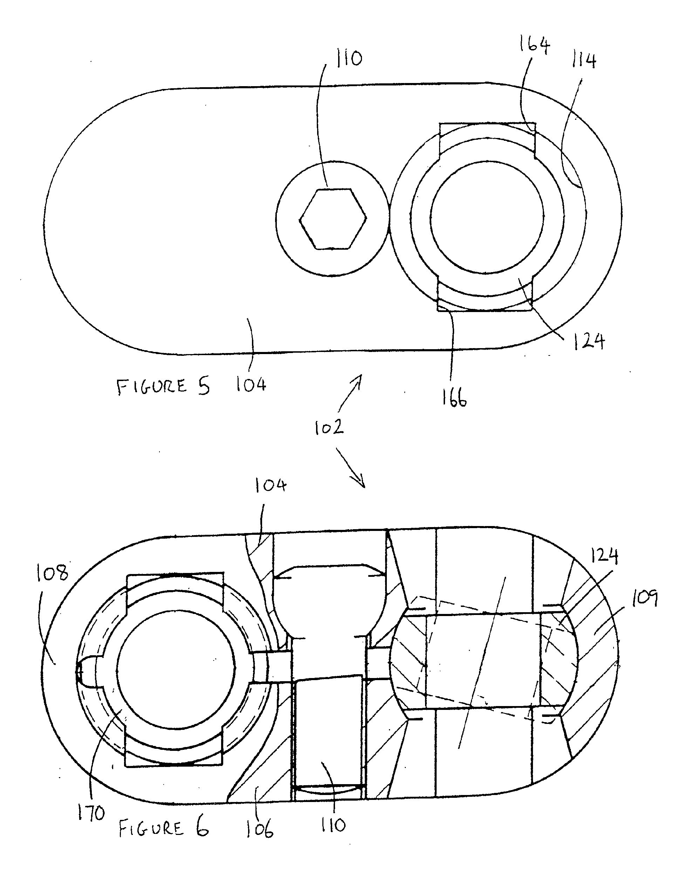

Referring to Figures 3 and 4, the second annular coupling 70 is provided with a spherical outer surface 74 and an opening 72 which extends along a central axis X-X of the second annular coupling 70. A slit 76 extends through the full radial and axial widths W1, W2 of the annulus of the annular coupling 70. Furthermore, a plurality of slots 78 extend through the full radial width W1 of the annulus of the annular coupling 70 but do not extend for the full axial width W2.

In operation of the swivel coupling 1, with the machine screw 10 loose, a spinal correction rod (not shown) is inserted into the opening 72 formed through the second annular coupling 70, and the collet portion 30 of the bone hook 32 is inserted into the opening 28 formed in the first annular coupling 24.

With the machine screw 10 loose, the spinal correction rod and bone hook 32 can be angled relative to one another by swivelling the annular couplings 24 and 70 in their respective openings 28, 72 in the swivel coupling 1. When the desired angular position has been found, the machine screw 10 is tightened, thereby bringing together the first clamping element 4 and second clamping element 6 and fixing the first annular coupling 24 and second annular coupling 70 in position relative to one another and relative to the main body 2.

It will be appreciated that as the machine screw 10 is tightened, and the first and second clamping elements 4, 6 are brought together, the second annular coupling 70, which has the form of a collet, is itself compressed, so that the opening 72 through the second annular coupling 70 is reduced in diameter, thereby gripping firmly the spinal correction rod and preventing it moving in an axial direction along the axis X-X of the second annular coupling 70.

Once the desired degree of insertion of the collet portion 30 of the bone hook 32 into the first annular coupling 24 has been determined, its position can be fixed by screwing the grub screw 58 into the threads of the collet portion 30. As the grub screw 58 is driven down into the bore 34 the tapered tip portion 62 of the grub screw 58 forces the tangs 42, 44, 46, 48 of the collet portion 30 outwardly into engagement with an inside surface of the opening 28 formed through the annular coupling 24.

If, inter-operatively or post-operatively, it is necessary to adjust the position of the bone hook 32 and spinal correction rod, the machine screw 10 and the grub screw 58 can be loosened, the various components repositioned and then the machine screw 10 and the bone screw 58 re-tightened. It is therefore very easy for a surgeon repeatedly to fix and release the relative positions of the bone hook 32, the body 2 and the spinal correction rod until the best orientation has been achieved.

Figures 5 and 6 show a second embodiment of swivel coupling 1 in which the first and second clamping elements 104, 106 are interconnected at both ends 108, 109. As best shown in Figure 5, the first annular coupling 124 is located in a first cavity 114 which is provided with rectangular recesses 164, 166 which are substantially identical to the recesses 64, 66 of the second cavity 16 of the first embodiment. The purposes of these cavities is to enable the first swivel coupling 124 to be inserted into the body portion 102. This is achieved by rotating the first swivel coupling 124 through 90°, relative to its orientation illustrated in Figure 5, pushing it along the recesses into a central region of the body portion 102 and then rotating the first annular coupling 124 through 90° to lock it into position. In the first embodiment of the invention, it is not necessary to provide this facility for the first annular coupling 24, as the first and second clamping elements are only interconnected at the first end 8 of the main body, and it is possible to insert the first annular coupling 24 by forcing the first and second clamping elements 4, 6 apart.

Although the present invention has been described in relation to spinal correction rods and bone engaging elements such as screws and hooks, it is applicable both to implanted and external fixation devices and could be used to attach any element of an internal or external fixation device to another element in which selective fixation of those components in relative angular orientations is desired.

Furthermore, it is contemplated that the swivel coupling may contain only a single annular coupling or may contain any number of couplings and may be interconnected and fixed by any combination and number of fixation devices such as screws, bolts, clips etc.

## Claims

1. A swivel coupling for attachment to human or animal bone, the device comprising a main body and a first connection element, the main body comprising first and second clamping elements and tightening means for tightening the clamping elements together, the first connection element being situated between the clamping elements, such that in a relaxed state of the tightening means, the first connection element is able to swivel relative to the body portion, whereas when the tightening element is tightened, the clamping elements are brought together to clamp the first connection element in a fixed position relative to the body portion.

2. A swivel coupling as claimed in claim 1, in which the first and second clamping elements are fixed together at an end of the main body.

3. A swivel coupling as claimed in claim 1, in which the first and second clamping elements are fixed together at both ends of the main body, but are spaced apart in between the said ends.

4. A swivel coupling as claimed in claim 2 or 3, in which the first and second clamping elements are integrally formed.

5. A swivel coupling as claimed in any one of the preceding claims, in which the first connection element is at least partially spherical.

6. A swivel coupling as claimed in any one of the preceding claims, in which the tightening means comprises a screw, which is engaged in a thread formed in the second clamping element, and which bears on the first clamping element with the underside of its head.

7. A swivel coupling as claimed in any one of the preceding claims, in which the first connection element is provided with an opening with receives a first substantially tubular member.

8. A swivel coupling as claimed in claim 7, in which the first substantially tubular member comprises a bone engaging element.

9. A swivel coupling as claimed in any one of the preceding claims, further comprising a second connection element which is situated between the first and second clamping elements and is clamped in position relative to the main body and the first connection element when the tightening element is tightened.

10. A swivel coupling as claimed in claim 9, in which the tightening element is situated between the first connection element and the second connection element.

11. A swivel coupling as claimed in claim 9 or 10, in which the second connection element is provided with a second opening which receives a second substantially tubular member.

12. A swivel coupling as claimed in claim 11, in which the second substantially tubular member comprises a spinal correction rod.

13. A swivel coupling as claimed in claim 11 or 12, when appendant to claim 9 or 10, in which a central axis of the opening in the first connection element is not parallel to the central axis of the opening in the second connection element.

14. A swivel coupling as claimed in any one of claims 11 to 13, in which the second connection element is split, so that as the clamping elements are tightened on to the second connection element, the opening through the second connection element is reduced in diameter, thereby to provide a clamping action.

15. A swivel coupling as claimed in any one of claims 11 to 14, in which the second connection element is provided with at least one slot which extends from the central opening to an outer surface of the second connection element.

16. A swivel coupling as claimed in any one of claims 9 to 15, in which the second connection element is at least partially spherical.

17. A device for attachment to human or animal bone comprising a bone engaging element and a connection element, the bone engaging element comprising a split collet, the open end of the collet being provided with an at least partially tapered bore in which is a located a threaded element, the split collet being received within an opening formed in the connection element, the threaded element being screwed into the open end of the collet to splay the collet into contact with an internal wall of the opening formed in the connection element.
